(19) Europäisches Patentamt / European Patent Office / Office européen des brevets

(11) **EP 2 114 387 B1**

(12) # FASCICULE DE BREVET EUROPEEN

(45) Date de publication et mention de la délivrance du brevet:
**06.04.2016 Bulletin 2016/14**

(21) Numéro de dépôt: **08761756.9**

(22) Date de dépôt: **11.01.2008**

(51) Int Cl.:
***A61K 31/05*** (2006.01) ***A61K 8/34*** (2006.01)
***A61K 36/575*** (2006.01)

(86) Numéro de dépôt international:
**PCT/FR2008/000035**

(87) Numéro de publication internationale:
**WO 2008/102086 (28.08.2008 Gazette 2008/35)**

(54) **UTILISATION DE COMPOSITIONS COMPRENANT AU MOINS UNE LIGNANE ET/OU NEOLIGNANE POUR MODULER LE TAUX DE TESTOSTERONE**

VERWENDUNG VON ZUSAMMENSETZUNGEN MIT MINDESTENS EINEM LIGNAN UND/ODER NEOLIGNAN ZUR MODULATION DER TESTOSTERON-SPIEGEL

USE OF COMPOSITIONS CONTAINING AT LEAST ONE LIGNANE AND/OR NEOLIGNANE FOR MODULATING TESTOSTERONE LEVELS

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **19.01.2007 FR 0700365**

(43) Date de publication de la demande:
**11.11.2009 Bulletin 2009/46**

(73) Titulaire: **Europe Finances**
**75016 Paris (FR)**

(72) Inventeurs:
- **BERNARD, Philippe**
  **45240 La Ferté Saint Aubin (FR)**
- **RASE, Didier**
  **F-75016 Paris (FR)**

(74) Mandataire: **Verriest, Philippe et al**
**Cabinet Germain & Maureau**
**12, rue Boileau**
**69006 Lyon (FR)**

(56) Documents cités:
**US-A1- 2003 224 064** **US-A1- 2006 251 608**
**US-A1- 2007 166 255** **US-B1- 6 338 855**

- **DATABASE WPI Week 199217 Thomson Scientific, London, GB; AN 1992-138627 XP002447456 & JP 04 082830 A (MIKIMOTO SEIYAKU KK) 16 mars 1992 (1992-03-16)**
- **MATSUDA HISASHI ET AL: "Effects of constituents from the bark of Magnolia obovata on nitric oxide production in lipopolysaccharide-activated macrophages" CHEMICAL AND PHARMACEUTICAL BULLETIN (TOKYO), vol. 49, no. 6, juin 2001 (2001-06), pages 716-720, XP001207307 ISSN: 0009-2363**
- **LEE ET AL: "Anti-inflammatory effects of magnolol and honokiol are mediated through inhibition of the downstream pathway of MEKK-1 in NF-.kappa.B activation signaling" PLANTA MEDICA, THIEME, STUTTGART, DE, vol. 71, no. 4, 2005, pages 338-343, XP009088409 ISSN: 0032-0943**
- **MARUYAMA Y ET AL: "OVERVIEW OF THE PHARMACOLOGICAL FEATURES OF HONOKIOL" CNS DRUG REVIEWS, BRANFORD, CT, US, vol. 6, no. 1, 2000, pages 35-44, XP001098195 ISSN: 1080-563X**

EP 2 114 387 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention concerne l'utilisation de compositions comprenant à titre d'agent actif au moins une lignane et/ou neolignane ou au moins un extrait végétal contenant une de ces lignanes et/ou neolignanes dans le domaine cosmétique, pour traiter et/ou prévenir des des troubles esthétiques résultant d'un processus de variation anormale du taux de testostérone.

Ces composés sont connus traditionnellement en Chine et au japon pour soigner notamment les troubles digestifs et les troubles de l'anxiété. Dès lors, de nombreuses recherches ont permis d'attribuer diverses propriétés biologiques à ces composés. Citons à titre d'exemple, des propriétés antibactériennes, anticarcinogènes, antiémétiques, antifongiques, antioxydantes, antiradicalaires, ou encore antiseptiques.

Des lignanes et/ou neolignanes ont été utilisés pour la préparation de compositions cosmétiques. On connaît, par exemple, de la demande de brevet chinoise CN1748673 des compositions pour traiter des désordres cerebro-cardiovasculaires, des demandes coréenne KR2006014203 et US20060140885 les propriétés anti-inflammatoires de ces composés et leurs applications respectivement en tant qu'inhibiteur de la génération de cytokines pro-inflammatoires produites par *Propionibacterium* pour le traitement de l'acné et en tant qu'ingrédient actif sous forme d'extraits de magnolia pour la préparation de formulations anti-inflammatoires orales.

Divers brevets ou demandes de brevet telles que le brevet US 6,338,855 ou les demandes de brevet US 2006/251608 et JP 04 082830 divulguent l'utilisation de lignane et/ou neolignanes comme agent anti-rides ou pour le traitement de l'acné. Néanmoins, de telles utilisations peuvent être liées à d'autres propriétés des lignanes et/ou néolignanes telles que les propriétés anti-inflammatoire et anti-oxydantes largement décrites dans la littérature. Aucune mention n'est faite dans ces documents d'une éventuelle activité des lignanes et/ou néolignanes sur le taux de testostérone.

Les travaux de recherche réalisés dans le cadre de la présente invention ont mis en évidence, de façon surprenante, que les lignanes et/ou néolignanes, notamment les lignanes et/ou néolignanes de type honokiol ou magnolol, permettaient de moduler le taux de testostérone, c'est-à-dire augmenter ou diminuer celui-ci selon le besoin.

Les lignanes et/ou neolignane de type honokiol et magnolol répondent à la structure chimique générale suivante (voir IUPAC Recommendations, Pure Applied Chemistry, Vol 72, n° 8, pp 1493-1523, 2000):

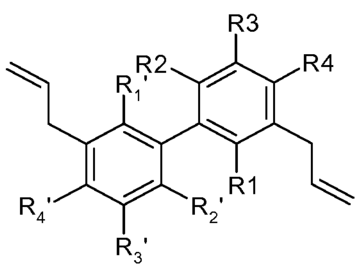

(I)

dans laquelle $R_1$ à $R_4$ et $R_{1'}$ à $R_{4'}$ identiques ou différents représentent chacun :

- soit un atome d'hydrogène,
- soit un atome d'halogène,
- soit un groupe $OR_x$, $SR_x$ ou $NR_xR_y$ dans lequel (i) $R_x$ et $R_y$, indépendamment l'un de l'autre, sont choisis parmi l'atome d'hydrogène, les groupes $(C_1\text{-}C_6)$alkyle, $(C_3\text{-}C_6)$cycloalkyle, $(C_6\text{-}C_{18})$aryle, $(C_6\text{-}C_{18})$aryle$(C_1\text{-}C_4)$alkyle, $(C_1\text{-}C_{12})$alkyle$(C_6\text{-}C_{18})$aryle, $(C_3\text{-}C_6)$cycloalkyle$(C_6\text{-}C_{12})$aryle, $(C_5\text{-}C_{12})$hétéroaryle comportant 1 à 3 hétéroatomes, NR'R" et NHCOR'R", R' et R", indépendamment l'un de l'autre, étant choisis parmi l'atome d'hydrogène et les groupes $(C_1\text{-}C_6)$ alkyle, $(C_3\text{-}C_6)$ cycloalkyle, $(C_6\text{-}C_{12})$aryle, et les hétérocycles en $(C_5\text{-}C_{12})$, aromatiques ou non, comportant 1 à 3 hétéroatomes ou (ii) $R_x$ et $R_y$ forment ensemble une chaîne hydrocarbonée linéaire ou ramifiée ayant de 2 à 6 atomes de carbone, comportant éventuellement une ou plusieurs doubles liaisons et/ou éventuellement interrompues par un atome d'oxygène, de soufre ou d'azote,
- un groupe $OR_x$, dans lequel $R_x$ correspond à un acide gras saturé ou insaturé $(C_4\text{-}C_{20})$, un ose ou un acide-aminé.

Les lignanes et/ou neolignanes de type honokiol ou magnolol sont présentes dans de nombreux végétaux, parmi lesquels on peut citer les végétaux appartenant à la famille des Magnoliacées comme le genre *Magniola* et à la famille des Lauracées, comme le genre *Sassafras.*

*Le magnolia* est une plante largement répandue comptant de nombreuses espèces. Elles ont déjà largement

été décrites dans la littérature pour de nombreuses propriétés biologiques. On attribue effectivement énormément de vertus à ces plantes sans pour autant en avoir démontré réellement et systématiquement l'efficacité.

La demande de brevet japonais JP 04/082830 décrit une composition utile pour le traitement de l'acné, comprenant du magnolol. Un exemple spécifique d'une telle composition comprenant 1% en poids de magnolol est également décrit. Cependant, aucune mention n'est faite d'une éventuelle action du magnolol sur le taux de testostérone. D'autre part, cette demande de brevet demeure silencieux quant à la possible utilisation cosmétique du magnolol pour prévenir ou traiter la chute des cheveux ou pour régénérer la matrice extracellulaire cutanée.

La demande de brevet américain US 2006/251608 divulgue une méthode de traitement de la peau abîmée/ridée/âgée comprenant

- ➢ l'administration orale d'au moins deux compositions,
- ➢ combinée à l'application topique d'une troisième composition.

Cette demande décrit dans son exemple 2 une composition comprenant moins de 1% d'honokiol, ladite composition se présentant sous la forme d'une gélule. La composition décrite dans l'exemple 2 n'est donc pas adaptée pour une application topique. En outre, aucune mention n'est faite dans ce document d'une action éventuelle de cet extrait sur le taux de testostérone ni de la possible utilisation d'un tel extrait pour prévenir ou traiter la chute des cheveux.

Dans le cadre de la présente invention, il a été mis en évidence que les lignanes et/ou néolignanes définies ci-dessus agissaient, de manière significative, comme inhibiteurs de l'aromatase, de la « Sex Hormone Binding Globulin » (SGHB) et de la 5-$\alpha$ réductase de type I, et confèrent *in vivo* une modulation du taux de testostérone.

L'aromatase est une enzyme qui dégrade la testostérone. Son inhibition permet d'empêcher la diminution du taux de testostérone.

La SHBG est une protéine de transport qui achemine les hormones sexuelles, dont la testostérone, dans les différents compartiments cellulaires. Lorsque la testostérone se lie à la SHBG, elle n'est plus disponible et n'est lentement relarguée que dans certaines conditions. L'inhibition de cette protéine permet d'augmenter le taux de testostérone libre.

La 5-$\alpha$ réductase de type I est une enzyme qui convertit la testostérone en composé actif dihydrotestostérone. Deux formes d'enzymes existent: la 5-$\alpha$ réductase de type I et la 5-$\alpha$ réductase de type II. Les inhibiteurs de 5-$\alpha$ réductase sont des médicaments utilisés pour traiter les problèmes d'hyperplasie prostatique et d'alopécie. Actuellement, la sélectivité entre les inhibiteurs de l'une ou l'autre des deux formes n'est pas clairement comprise pour traiter les problèmes d'alopécie. De plus, il n'existe pas d'inhibiteurs sélectif de 5-$\alpha$ réductase de type I.

Ces processus ont été largement étudiés ces dernières années, car ils sont à l'origine de nombreux intérêts thérapeutiques notamment dans le cas du traitement des cancers de la prostate. A titre d'exemple, divers principes actifs médicamenteux connus sous les noms de letrozole ou d'anastrozole sont actuellement sur le marché comme inhibiteurs d'aromatase.

Il a été découvert dans le cadre de la présente invention que lesdites lignanes et/ou néo-lignanes en application topique ont un effet modulateur sur le taux de testostérone.

Plus particulièrement, il a été découvert, de façon toute à fait surprenante, que :

- les compositions comprenant entre 0 et 2% (préférentiellement entre 0 et 1%) en poids par rapport au poids total de la composition desdites lignanes et/ou néo-lignanes stimulent la production de testostérone;
- au contraire, les compositions comprenant de 2 à 4% en poids par rapport au poids total de la composition desdites lignanes et/ou néo-lignanes inhibent la production de testostérone.
- enfin, les compositions comprenant plus de 4% (de préférence entre 4% et 5%) en poids par rapport au poids total de la composition de lignanes et/ou néo-lignanes stimulent à nouveau la production de testostérone.

Comme indiqué précédemment, la Demanderesse a mis en évidence que les lignanes et/ou néolignanes, et plus particulièrement l'honokiol et le magnolol, permettent à des concentrations très précises de restaurer le taux de testostérone, par exemple chez les personnes atteintes de dysfonctionnement des hormones sexuelles.

De telles compositions peuvent ainsi être utilisées pour traiter et/ou prévenir des troubles esthétiques résultant de cette chute du taux de testostérone. Ainsi, de telles compositions sont particulièrement adaptées à une application topique pour prévenir et/ou traiter les altérations cutanées résultant de la chute du taux de testostérone telle que la dégradation de la matrice extracellulaire cutanée. En effet, l'application de telles compositions permet de une redensification du derme et de redonner un caractère souple, résistant et élastique à la peau.

De telles compositions peuvent également être utilisées pour prévenir et/ou traiter la chute de cheveux.

La présente invention a donc pour objet l'utilisation cosmétique pour une application topique d'une composition comprenant 1% ou moins en poids par rapport au poids total de la composition d'un ou plusieurs composés de formule (I) :

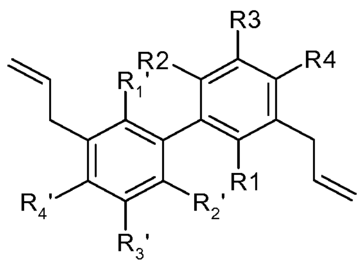

(I)

dans laquelle $R_1$ à $R_4$ et $R_{1'}$ à $R_{4'}$, identiques ou différents, représentent chacun :

- soit un atome d'hydrogène,
- soit un atome d'halogène,
- soit un groupe $OR_x$, $SR_x$ ou $NR_xR_y$ dans lequel (i) $R_x$ et $R_y$, indépendamment l'un de l'autre, sont choisis parmi l'atome d'hydrogène, les groupes $(C_1-C_6)$alkyle, $(C_3-C_6)$cycloalkyle, $(C_6-C_{18})$aryle, $(C_6-C_{18})$aryle$(C_1-C_4)$alkyle, $(C_1-C_{12})$alkyle$(C_6-C_{18})$aryle, $(C_3-C_6)$cycloalkyle$(C_6-C_{12})$aryle, $(C_5-C_{12})$hétéroaryle comportant 1 à 3 hétéroatomes, NR'R" et NHCOR'R", R' et R", indépendamment l'un de l'autre, étant choisis parmi l'atome d'hydrogène et les groupes $(C_1-C_6)$ alkyle, $(C_3-C_6)$ cycloalkyle, $(C_6-C_{12})$aryle, et les hétérocycles en $(C_5-C_{12})$, aromatiques ou non, comportant 1 à 3 hétéroatomes ou (ii) $R_x$ et $R_y$ forment ensemble une chaîne hydrocarbonée linéaire ou ramifiée ayant de 2 à 6 atomes de carbone, comportant éventuellement une ou plusieurs doubles liaisons et/ou éventuellement interrompues par un atome d'oxygène, de soufre ou d'azote,
- un groupe $OR_x$, dans lequel $R_x$ correspond à un acide gras saturé ou insaturé $(C_4-C_{20})$, un ose ou un acide-aminé ;

ou leurs sels ou complexes cosmétiquement acceptables,
pour traiter ou prévenir une dégradation de la matrice extracellulaire cutanée résultant d'une chute du taux de testostérone.

La présente invention a également pour objet l'utilisation cosmétique d'une composition comprenant de 2% à 4% en poids par rapport au poids total de la composition d'un ou plusieurs composés de formule (I) :

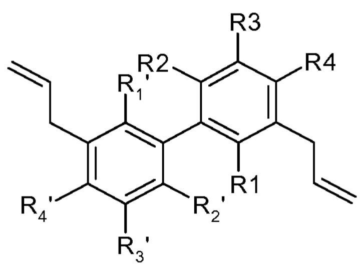

(I)

dans laquelle $R_1$ à $R_4$ et $R_{1'}$ à $R_{4'}$, identiques ou différents, représentent chacun :

- soit un atome d'hydrogène,
- soit un atome d'halogène,
- soit un groupe $OR_x$, $SR_x$ ou $NR_xR_y$ dans lequel (i) $R_x$ et $R_y$, indépendamment l'un de l'autre, sont choisis parmi l'atome d'hydrogène, les groupes $(C_1-C_6)$alkyle, $(C_3-C_6)$cycloalkyle, $(C_6-C_{18})$aryle, $(C_6-C_{18})$aryle$(C_1-C_4)$alkyle, $(C_1-C_{12})$alkyle$(C_6-C_{18})$aryle, $(C_3-C_6)$cycloalkyle$(C_6-C_{12})$aryle, $(C_5-C_{12})$hétéroaryle comportant 1 à 3 hétéroatomes, NR'R" et NHCOR'R", R' et R", indépendamment l'un de l'autre, étant choisis parmi l'atome d'hydrogène et les groupes $(C_1-C_6)$ alkyle, $(C_3-C_6)$ cycloalkyle, $(C_6-C_{12})$aryle, et les hétérocycles en $(C_5-C_{12})$, aromatiques ou non, comportant 1 à 3 hétéroatomes ou (ii) $R_x$ et $R_y$ forment ensemble une chaîne hydrocarbonée linéaire ou ramifiée ayant de 2 à 6 atomes de carbone, comportant éventuellement une ou plusieurs doubles liaisons et/ou éventuellement interrompues par un atome d'oxygène, de soufre ou d'azote,
- un groupe $OR_x$, dans lequel $R_x$ correspond à un acide gras saturé ou insaturé $(C_4-C_{20})$, un ose ou un acide-aminé ;

ou leurs sels ou complexes cosmétiquement acceptables,
pour traiter ou prévenir la chute de cheveux.

Dans un mode de réalisation préféré, le composé de formule générale (I) est choisi comme étant l'honokiol et/ou le magnolol de formule (II) et (III) respectivement ci-dessous :

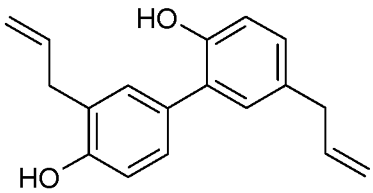

(II)

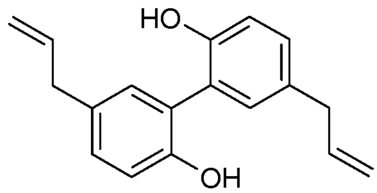

(III)

Dans un autre mode de réalisation les composés sont choisis dans le groupe constitué par le Caryolanemagnolol, le Clovanemagnolol, le Dehydrodieugenol et ses dérivés Méthyle éther, le Dunnianiol et ses dérivés, l'Eudeshonokiol A et B, l'Eudesmagnolol, l'Isodunnianol, le Magnoligan F, le Peltatol A, le Piperitylmagnolol et ses dérivés.

Préférentiellement, la présente invention concerne lesdites utilisations caractérisées en ce que ladite compositioncosmétique comprend en outre un véhicule compatible et approprié au mode d'administration choisi.

Dans un mode de réalisation, les composés sont choisis parmi les sels ou complexes cosmétiquement acceptables des composés ci-dessus cités, tels que les sels formés avec des acides inorganiques ou les sels formés avec des acides organiques.

Parmi les sels formés avec des acides inorganiques selon l'invention, on peut citer à titre d'exemple les sels formés avec les acides choisis dans le groupe comprenant les acides chlorhydrique, bromhydrique, sulfurique, phosphorique et/ou nitrique.

Parmi les sels formés avec des acides organiques selon l'invention on peut citer à titre d'exemple les sels formés avec les acides choisis dans le groupe comprenant les acides acétique, oxalique, tartrique, succinique, malique, fumarique, maléique, ascorbique, benzoique, tannique, alginique, polyglutamique, naphtalène sulfonique, naphtalène disulfonique et/ou polygalacturonique.

Dans un mode de réalisation les lignanes et/ou neolignanes sont choisies parmi les composés extraits des écorces, fleurs, graines et/ou racines de végétaux appartenant à la famille des Magnoliacées comme le genre *Magniolia.*

Dans un mode de réalisation les végétaux sont choisis dans la famille des Lauracées, comme le genre *Sassafras.*

Dans un mode de réalisation les végétaux sont choisis dans le groupe des plantes comprenant les genres *Illicium, Sassafras, Potomorphe, Nectandra, Ocotea, Virola* et/ou *Litsea.*

Dans un mode de réalisation les lignanes et/ou neolignanes sont apportés par l'huile extraite des feuilles de *Cymbopogon winterianus* (Java citronella oil).

Les lignanes et/ou néolignanes selon la présente invention, et plus particulièrement l'honokiol et le magnolol, et les extraits végétaux les contenant, peuvent être associés dans les compositions destinées à l'utilisation selon l'invention avec d'autres composés présentant des propriétés diverses. A titre d'exemple mais de manière non limitative, on peut citer les associations avec des mucopolysaccharides, des vitamines, des céramides, des substances antiradicalaires, des filtres U.V., des antioxydants ainsi que d'autres actifs.

Les compositions cosmétiques destinées à l'utilisation selon l'invention peuvent se présenter sous la forme de crèmes, gels, lotions, laits, émulsions H/E et E/H, solutions, onguents, pulvérisateurs, huiles corporelles, lotions capillaires, shampooings, lotions après-rasage, savons, bâtons protecteur des lèvres, bâtons et crayons pour maquillage.

Sous la forme de gel, elles comprennent des excipients appropriés tels que les esters de cellulose ou d'autres agents gélifiants, tels que le carbopol, la gomme guar.

Ces compositions cosmétiques peuvent aussi prendre la forme de lotion ou solution dans laquelle les extraits et/ou molécules sont sous forme encapsulée, par exemple dans des microsphères. Ces microsphères peuvent par exemple être constituées de corps gras, d'agar et d'eau. Les agents actifs peuvent être aussi incorporés dans des vecteurs de type liposomes, glycosphères, dans des chylomicrons, des macro-, micro-, nano-particules ainsi que les macro-, micro- et nanocapsules et aussi être adsorbés sur des polymères organiques poudreux, les talcs, bentonites et autres supports minéraux. Ces émulsions jouissent d'une bonne stabilité et peuvent être conservées pendant le temps nécessaire pour l'utilisation à des températures comprises entre 0 et 50 °C sans qu'il y ait sédimentation des constituants ou séparation des phases.

Les compositions cosmétiques de l'invention comprennent entre 0,1 à 1% en poids d'agents actifs lorsqu'elles sont sous forme de poudre ou sous forme encapsulée.

Pour la préparation de ces compositions, la néolignane, et plus particulièrement l'honokiol ou le magnolol, ou

un extrait végétal, sont mélangés aux excipients généralement employés en cosmétique.

Les compositions cosmétiques destinées à l'utilisation selon l'invention peuvent aussi contenir des additifs ou des adjuvants usuels en cosmétologie, comme par exemple des agents antibactériens ou des parfums mais aussi des lipides d'extraction et/ou de synthèse, polymères gélifiants et viscosants, tensio-actifs et émulsifiants, principes actifs hydro ou liposolubles, extraits de plantes, extraits tissulaires, extraits marins, actifs de synthèse.

L'utilisation cosmétique ou dermocosmétique d'extraits et/ou molécules comprend tous les soins du corps et de la peau y compris les produits solaires, protecteurs et bronzants, les produits anti-âge, anti-seborrhéïques, toniques, les produits assurant l'amélioration de l'aspect de la peau y compris le traitement acnéique, les rougeurs cutanées, le traitement du cuir chevelu et celui de la chute des cheveux.

Les compositions cosmétiques destinées à l'utilisation selon l'invention peuvent aussi comprendre d'autres principes actifs complémentaires choisis pour leur action, par exemple pour la protection solaire, l'effet anti-rides, l'activité antiradicalaire et antioxydante, l'activité anti-irritante, la nutrition cellulaire, la respiration cellulaire, l'hydratation et la régénération cellulaire, les traitements anti-séborrhéïques, anti-acnéiques ainsi que d'autres principes actifs ayant une action sur la tonicité cutanée, la protection du cheveu.

Les compositions cosmétiques destinées à l'utilisation selon l'invention sont de préférence à utiliser quotidiennement en les appliquant une ou plusieurs fois par jour.

Les compositions cosmétiques destinées à l'utilisation selon l'invention sont très bien tolérées, elles ne présentent aucune phototoxicité et leur application sur la peau, pour des périodes de temps prolongées, n'implique aucun effet systémique.

Ces compositions dermocosmétiques se présentent sous forme liquide, de poudre, de pâte ou d'émulsion, seule ou en combinaison avec d'autres substances. Elles comprennent de l'ordre de 0,1 à 5% en poids de néolignane, et plus particulièrement d'honokiol ou de magnolol, ou d'un extrait végétal les contenant.

Les exemples qui suivent illustrent l'activité de l'honokiol, dans des tests d'activité *in vivo* et dans des tests de toxicité. Des résultats similaires ont été obtenus avec d'autres dérivés.

**Exemple 1 : Détermination du taux de testostérone en fonction de la dose administrée en principes actifs**

Introduction

Cette étude vise à déterminer les effets *in vivo* de l'honokiol (0.5, 1, 5, 10, 15 et 25 mg/kg) en fonction de la dose administrée, cela comparé au letrozole (2 mg/kg) sur le taux plasmatique de testostérone chez la souris SWISS.

L'effet du letrozole est déterminé en administration sub-chronique (trois administrations journalières) chez des souris adultes mâles.

Les effets du letrozole et de l'honokiol sont déterminés en administration chronique (10 à 14 administrations journalières) sur le taux plasmatique de testostérone chez des souris mâles jeunes.

Matériels et méthodes

Animaux

- Soixante quatre souris Swiss RjOrl (Centre d'Elevage René Janvier, France) :
    - ◦ Etude du letrozole et de l'honokiol : 64 souris mâles âgées de 3 semaines en début de traitement et de 5 semaines en fin de traitement.

Produits

- Letrozole
    - Dose étudiée : 2 mg/kg.
    - Mise en solution extemporanée dans le méthylcellulose
- Honokiol

- Doses étudiée :
    - Honokiol : 0.01, 0.05, 0.5, 2, 5, 10 mg/kg.
- Mise en solution extemporanée dans le méthylcellulose

- Voie d'administration : intra-péritonéale (i.p.).
- Volume d'injection : 10 ml/kg.

Dosages

Les dosages ont été réalisés selon le protocole du kit ELISA commercialisé par la société AbCys S.A., France.

Résultats - Conlusion

Les résultats sont présentés en figure 1. qui illustre le taux de testostérone plasmatique en fonction des concentrations en letrozole et en Honokiol administrées. On observe une modulation du taux de testostérone en fonction des doses administrées, l'augmentation étant dose dépendante avec un effet de seuil.

Comparative

Une préparation dermatologique titrée entre 2% et 4% en Honokiol et dérivés présente des effets significatifs dans le traitement de l'acné, maladie topique liée à un trouble du métabolisme hormonal.

**Exemple 3 : Etude clinique d'un produit titré à 1% en Honokiol**

Etude de tolérance : L'étude patch test relative à l'évaluation de la tolérance cutanée aiguë de la matière première : crème titrée à 5% en Honokiol dans les conditions expérimentales retenues : 48 heures, occlusif, pur, 20 volontaires, peau normale), montre que cette matière première s'avère non irritante aux lectures 30 minutes et 24 heures.

Etude clinique :

Deux groupes de 15 hommes volontaires de 55 à 65 ans ont participé à l'étude clinique.
L'application biquotidienne a été suivie pendant 56 jours. Les mesures avant/après ont été réalisées.
Deux paramètres ont été mesurés : effet anti-rides et effet redensifiant du derme.
Dans les conditions expérimentales retenues:

*Effet anti-rides*

- Le produit a entraîné, après 28 jours d'utilisation, une diminution du nombre de sillons du microrelief (-13%) et du nombre de rides moyennes (-3%) chez 69% des volontaires. D'autre part, après 56 jours d'utilisation du produit, l'analyse des empreintes réalisées sur les pattes d'oie, montre une diminution significative du nombre de rides profondes (en moyenne : -11%).

Ces résultats sont caractéristiques d'une tendance à un effet anti-rides et à un effet lissant.
La figure 2 représente l'effet anti-rides du placebo (à gauche) contre le produit titré à 1% en Honokiol (à droite) selon la durée de traitement.

*Effet redensifiant*

- Le placebo (crème sans actif) n'a entraîné aucune variation significative de la densité du derme après 56 jours d'utilisation.
- Le produit (crème à 1 % en actif selon l'invention) a entraîné une augmentation de la densité du derme chez 60% des volontaires. En moyenne +5% de densité. La figure 3 montre l'effet redensifiant du produit sur le derme pour

le produit titré à 1% en Honokiol à J0 contre J56. L'effet redensifiant du derme et de reformation de la matrice extracellulaire est significatif.

En conclusion,

- les résultats du placebo ne montrent ni effet anti-rides ni effet redensifiant.
- les résultats du produit titré à 1% montrent un effet lissant et anti-rides ainsi qu'un effet redensifiant du derme.

**Exemple 4 : Inhibition des isoenzymes 5-alpha-réductases par l'honokiol et ses dérivés**

Culture cellulaire

Les cellules de type HEK I et HEK II ont été utilisées dans du DMEM (pH 7.4) avec 10 % FCS, Penicillin/Streptomycin (100 U/mL and 100 μg/mL) et 0.5 mg/mL de Geneticin-418-sulfate. Les tests d'inhibition ont été réalisés à des concentrations cellulaires de 0.25 x 106 cellules par 1.9 $cm^2$ et incubées pendant 20 h, 5 % CO2 à 37 °C.

Tests d'inhibition in vitro

Les composés sont dissous dans le DMSO et dilués avec le DMEM pour atteindre les concentrations finales de 100 μM, 10 μM et 1 μM.
Les mesures ont été réalisées grâce à un Phospholmager et les spots correspondant au [4-14C]-androstenedione (A) et au [4-14C]-dihydroandrostenedione (DHA) furent enregistrés avec le logiciel correspondant.

Analyses des données.

Les calculs ont été réalisés selon la formule suivante:

$$\% \text{ conversion} = \frac{\text{PSL(DHA)}}{\text{PSL(DHA + A)}} \times 100$$

Le pourcentage d'inhibition a été calculé sur une moyenne de deux mesures.

Résultats

Les résultats sur l'inhibition des 5α-reductase type I and type II sont présentés dans le tableau ci-dessous.
La référence est le finastéride, un médicament inhibiteur des 5α-reductase qui est utilisé comme contrôle positif à deux concentrations de 800 nM et 100 nM, correspondant à l'IC50 du finasteride pour la 5α-reductase de type I et la 5α-reductase de type II, respectivement.

| Composés | concentration | % inhibition de la 5-α reductase type I | % inhibition de la 5-α reductase type II |
|---|---|---|---|
| Honokiol | 1 μM | 1 | n.i. |
| | 10 μM | 11 | n.i. |
| | 100 μM | 64 | n.i. |
| Finasteride | 100 nM | 11 | 55 |
| | 800 nM | 48 | 89 |
| n.i.: pas d'inhibition observée | | | |

Il apparaît que le finastéride inhibe à la fois sur la 5-α réductase de type I et la 5-α réductase de type II.
Au contraire, l'honokiol inhibe de manière dose dépendante la 5α-réductase de type I avec une estimation de l'IC50 de l'ordre de 80 μM, mais aucune affinité n'est observée pour la 5-α réductase de type II.
En conclusion, il s'avère que l'honokiol est un inhibiteur sélectif de la 5-α réductase de type I ; ce qui est tout-à-fait inattendu et surprenant car il s'agirait d'une nouvelle classe original d'inhibiteur sélectif de cette isoenzyme.

## Revendications

1. Utilisation cosmétique pour une application topique d'une composition comprenant 1% ou moins en poids par rapport au poids total de la composition d'un ou plusieurs composés de formule (I) :

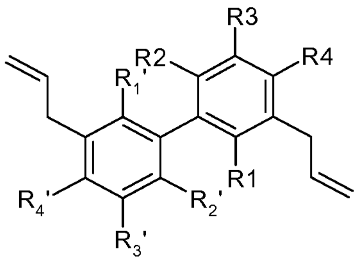

(I)

dans laquelle $R_1$ à $R_4$ et $R_{1'}$ à $R_{4'}$, identiques ou différents, représentent chacun :

- soit un atome d'hydrogène,
- soit un atome d'halogène,
- soit un groupe $OR_x$, $SR_x$ ou $NR_xR_y$ dans lequel (i) $R_x$ et $R_y$, indépendamment l'un de l'autre, sont choisis parmi l'atome d'hydrogène, les groupes ($C_1$-$C_6$)alkyle, ($C_3$-$C_6$)cycloalkyle, ($C_6$-$C_{18}$)aryle, ($C_6$-$C_{18}$)aryle($C_1$-$C_4$)alkyle, ($C_1$-$C_{12}$)alkyle($C_6$-$C_{18}$)aryle, ($C_3$-$C_6$)cycloalkyle($C_6$-$C_{12}$)aryle, ($C_5$-$C_{12}$)hétéroaryle comportant 1 à 3 hétéroatomes, NR'R" et NHCOR'R", R' et R", indépendamment l'un de l'autre, étant choisis parmi l'atome d'hydrogène et les groupes ($C_1$-$C_6$) alkyle, ($C_3$-$C_6$) cycloalkyle, ($C_6$-$C_{12}$)aryle, et les hétérocycles en ($C_5$-$C_{12}$), aromatiques ou non, comportant 1 à 3 hétéroatomes ou (ii) $R_x$ et $R_y$ forment ensemble une chaîne hydrocarbonée linéaire ou ramifiée ayant de 2 à 6 atomes de carbone, comportant éventuellement une ou plusieurs doubles liaisons et/ou éventuellement interrompues par un atome d'oxygène, de soufre ou d'azote,
- un groupe $OR_x$, dans lequel $R_x$ correspond à un acide gras saturé ou insaturé ($C_4$-$C_{20}$), un ose ou un acide-aminé ;

ou leurs sels ou complexes cosmétiquement acceptables,
pour traiter ou prévenir une dégradation de la matrice extracellulaire cutanée résultant d'une chute du taux de testostérone.

2. Utilisation cosmétique d'une composition comprenant de 2% à 4% en poids par rapport au poids total de la composition d'un ou plusieurs composés de formule (I) :

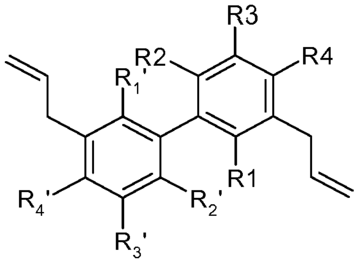

(I)

dans laquelle $R_1$ à $R_4$ et $R_{1'}$ à $R_{4'}$, identiques ou différents, représentent chacun :

- soit un atome d'hydrogène,
- soit un atome d'halogène,
- soit un groupe $OR_x$, $SR_x$ ou $NR_xR_y$ dans lequel (i) $R_x$ et $R_y$, indépendamment l'un de l'autre, sont choisis parmi l'atome d'hydrogène, les groupes ($C_1$-$C_6$)alkyle, ($C_3$-$C_6$)cycloalkyle, ($C_6$-$C_{18}$)aryle, ($C_6$-$C_{18}$)aryle($C_1$-$C_4$)alkyle, ($C_1$-$C_{12}$)alkyle($C_6$-$C_{18}$)aryle, ($C_3$-$C_6$)cycloalkyle($C_6$-$C_{12}$)aryle, ($C_5$-$C_{12}$)hétéroaryle comportant 1 à 3 hétéroatomes, NR'R" et NHCOR'R", R' et R", indépendamment l'un de l'autre, étant choisis parmi l'atome d'hydrogène et les groupes ($C_1$-$C_6$) alkyle, ($C_3$-$C_6$) cycloalkyle, ($C_6$-$C_{12}$)aryle, et les hétérocycles en ($C_5$-$C_{12}$), aromatiques ou non, comportant 1 à 3 hétéroatomes ou (ii) $R_x$ et $R_y$ forment ensemble une chaîne hydrocarbonée linéaire ou ramifiée ayant de 2 à 6 atomes de carbone, comportant éventuellement une ou plusieurs doubles liaisons et/ou éventuellement interrompues par un atome d'oxygène, de soufre ou d'azote,
- un groupe $OR_x$, dans lequel $R_x$ correspond à un acide gras saturé ou insaturé ($C_4$-$C_{20}$), un ose ou un acide-aminé ;

ou leurs sels ou complexes cosmétiquement acceptables,
pour traiter ou prévenir la chute de cheveux résultant d'un taux de testostérone trop élevé.

3. Utilisation selon la revendication 1 ou 2, **caractérisée en ce que** le composé de formule générale (I) est choisi comme étant l'honokiol ou le magnolol respectivement de formule (II) et (III) :

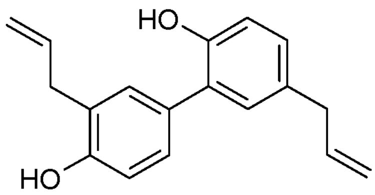

(II)

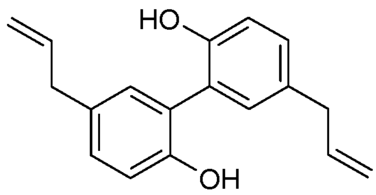

(III)

4. Utilisation selon l'une quelconque des revendications 1 à 3, **caractérisée en ce que** le ou les composés de formule générale (I) sont associés avec d'autres composés présentant des propriétés diverses tels que des mucopolysaccharides, des vitamines, des céramides, des substances antiradicalaires, des filtres U.V., des antioxydants ainsi que d'autres actifs.

**Patentansprüche**

1. Kosmetische Verwendung für eine topische Anwendung einer Zusammensetzung, die 1 Gew.-% oder weniger einer oder mehrerer Verbindungen der Formel (I) umfasst, bezogen auf das Gesamtgewicht der Zusammensetzung:

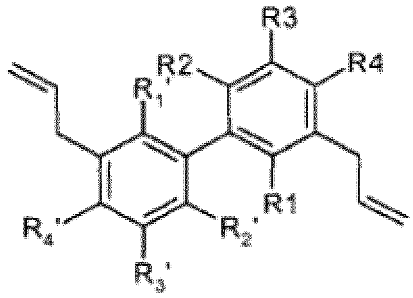

(I),

in der $R_1$ bis $R_4$ und $R_{1'}$ bis $R_{4'}$, die gleich oder verschieden sind, jeweils für folgendes stehen:

- entweder ein Wasserstoffatom,
- oder ein Halogenatom,
- oder eine Gruppe $OR_x$, $SR_x$ oder $NR_xR_y$, in der (i) $R_x$ und $R_y$ unabhängig voneinander aus einem Wasserstoffatom, den Gruppen ($C_1$-$C_6$)-Alkyl, ($C_3$-$C_6$)-Cycloalkyl, ($C_6$-$C_{18}$)-Aryl, ($C_6$-$C_{18}$)-Aryl-($C_1$-$C_4$)-alkyl, ($C_1$-$C_{12}$)-Alkyl-($C_6$-$C_{18}$)-aryl, ($C_3$-$C_6$)-Cycloalkyl-($C_6$-$C_{12}$)-aryl, ($C_5$-$C_{12}$)-Heteroaryl, das 1 bis 3 Heteroatome umfasst, NR'R" und NHCOR'R" ausgewählt sind, wobei R' und R" unabhängig voneinander aus einem Wasserstoffatom und den Gruppen ($C_1$-$C_6$)-Alkyl, ($C_3$-$C_6$)-Cycloalkyl, ($C_6$-$C_{12}$)-Aryl und gegebenenfalls aromati-

schen ($C_5$-$C_{12}$)-Heterocyclen, die 1 bis 3 Heteroatome umfassen, ausgewählt sind, oder (ii) $R_x$ und $R_y$ zusammen eine lineare oder verzweigte Kohlenwasserstoffkette mit 2 bis 6 Kohlenstoffatomen bilden, die gegebenenfalls eine oder mehrere Doppelbindungen umfasst und/oder gegebenenfalls durch ein Sauerstoff-, Schwefel- oder Stickstoffatom unterbrochen ist,
- eine Gruppe $OR_x$, in der $R_x$ einer gesättigten oder ungesättigten ($C_4$-$C_{20}$)-Fettsäure, einem Monosaccharid oder einem Säureamid entspricht;

oder von deren kosmetisch akzeptablen Salzen oder Komplexen,
zur Behandlung oder Verhinderung eines Abbaus der extrazellulären Matrix der Haut, der aus einem Rückgang des Testosterongehalts resultiert.

**2.** Kosmetische Verwendung einer Zusammensetzung, die 2 Gew.-% bis 4 Gew.-% einer oder mehrerer Verbindungen der Formel (I) umfasst, bezogen auf das Gesamtgewicht der Zusammensetzung:

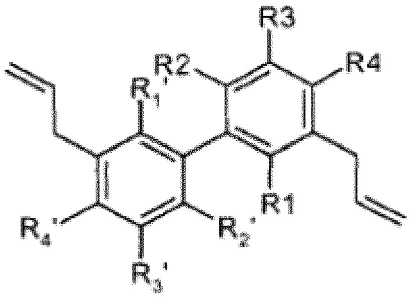

(I),

in der $R_1$ bis $R_4$ und $R_{1'}$ bis $R_{4'}$, die gleich oder verschieden sind, jeweils für folgendes stehen:

- entweder ein Wasserstoffatom,
- oder ein Halogenatom,
- oder eine Gruppe $OR_x$, $SR_x$ oder $NR_xR_y$, in der (i) $R_x$ und $R_y$ unabhängig voneinander aus einem Wasserstoffatom, den Gruppen ($C_1$-$C_6$)-Alkyl, ($C_3$-$C_6$)-Cycloalkyl, ($C_6$-$C_{18}$)-Aryl, ($C_6$-$C_{18}$)-Aryl-($C_1$-$C_4$)-alkyl, ($C_1$-$C_{12}$)-Alkyl-($C_6$-$C_{18}$)-aryl, ($C_3$-$C_6$)-Cycloalkyl-($C_6$-$C_{12}$)-aryl, ($C_5$-$C_{12}$)-Heteroaryl, das 1 bis 3 Heteroatome umfasst, NR'R" und NHCOR'R" ausgewählt sind, wobei R' und R" unabhängig voneinander aus einem Wasserstoffatom und den Gruppen ($C_1$-$C_6$)-Alkyl, ($C_3$-$C_6$)-Cycloalkyl, ($C_6$-$C_{12}$)-Aryl und gegebenenfalls aromatischen ($C_5$-$C_{12}$)-Heterocyclen, die 1 bis 3 Heteroatome umfassen, ausgewählt sind, oder (ii) $R_x$ und $R_y$ zusammen eine lineare oder verzweigte Kohlenwasserstoffkette mit 2 bis 6 Kohlenstoffatomen bilden, die gegebenenfalls eine oder mehrere Doppelbindungen umfasst und/oder gegebenenfalls durch ein Sauerstoff-, Schwefel- oder Stickstoffatom unterbrochen sind,
- eine Gruppe $OR_x$, in der $R_x$ einer gesättigten oder ungesättigten ($C_4$-$C_{20}$)-Fettsäure, einem Monosaccharid oder einem Säureamid entspricht;

oder von deren kosmetisch akzeptablen Salzen oder Komplexen,
zur Behandlung oder Verhinderung von Haarausfall, der aus einem zu hohen Testosterongehalt resultiert.

**3.** Verwendung nach Anspruch 1 oder 2, **dadurch gekennzeichnet, dass** die Verbindung der allgemeinen Formel (I) als Honokiol oder Magnolol der Formel (II) bzw. (III) ausgewählt ist:

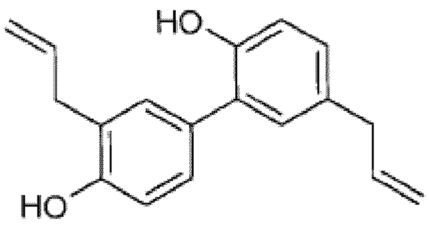

(II)

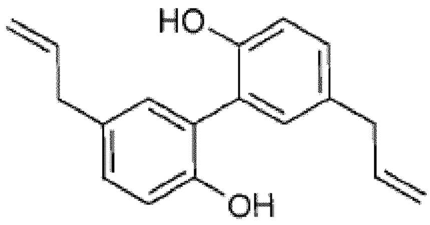

(III) .

4. Verwendung nach einem der Ansprüche 1 bis 3, **dadurch gekennzeichnet, dass** die Verbindung oder die Verbindungen der Formel (I) mit anderen Verbindungen vereinigt sind, die andere Eigenschaften aufweisen, wie Mukopolysaccharide, Vitamine, Ceramide, Radikalfänger, UV-Filter, Antioxidationsmittel sowie andere Wirkstoffe.

## Claims

1. A cosmetic use for topical application of a composition comprising 1 % or less by weight relative to the total weight of the composition of one or more compound(s) of formula (I):

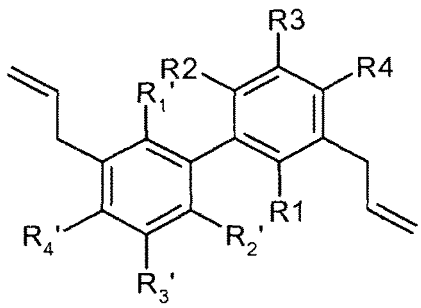

(I)

wherein $R_1$ to $R_4$ and $R_{1'}$ to $R_{4'}$, identical or different, represent each:

- either a hydrogen atom,
- or a halogen atom,
- or a group $OR_x$, $SR_x$ or $NR_xR_y$ wherein (i) $R_x$ and $R_y$ independently from each other, are selected from the hydrogen atom, the groups $(C_1-C_6)$alkyl, $(C_3-C_6)$cycloalkyl, $(C_6-C_{18})$aryl, $(C_6-C_{18})$aryl$(C_1-C_4)$alkyl, $(C_1-C_{12})$alkyl$(C_6-C_{18})$aryl, $(C_3-C_6)$cycloalkyl$(C_6-C_{12})$aryl, $(C_5-C_{12})$heteroaryl including 1 to 3 heteroatoms, NR'R" and NHCOR'R", R' and R", independently from each other, being selected from the hydrogen atom and the groups $(C_1-C_6)$alkyl, $(C_3-C_6)$cycloalkyl, $(C_6-C_{12})$aryl, and the $(C_6-C_{12})$heterocycles, aromatic or not, including 1 to 3 heteroatoms or (ii) $R_x$ and Ryform together a linear or branched hydrocarbon chain having from 2 to 6 carbon atoms, optionally including one or more double bond(s) and/or optionally interrupted by an oxygen, sulfur or nitrogen atom,
- an $OR_x$ group, wherein $R_x$ corresponds to a $(C_4-C_{20})$ saturated or unsaturated fatty acid, a monosaccharide or an amino acid;

or their cosmetically acceptable salts or complexes,
to treat or prevent a degradation of the dermal extracellular matrix resulting from a drop in testosterone level.

2. The cosmetic use of a composition comprising from 2% to 4% by weight relative to the total weight of the composition of one or more compound(s) of formula (I):

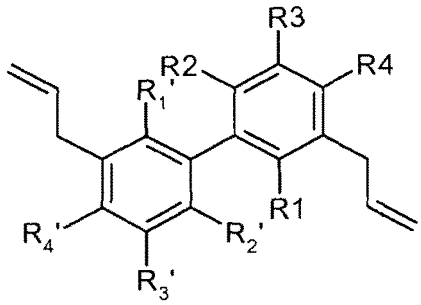

(I)

wherein $R_1$ to $R_4$ and $R_{1'}$ to $R_{4'}$ identical or different, represent each:

- either a hydrogen atom,
- or a halogen atom
- or a group $OR_x$, $SR_x$ or $NR_xR_y$ wherein (i) $R_x$ and $R_y$, independently from each other, are chosen from the hydrogen atom, the groups $(C_1\text{-}C_6)$alkyl, $(C_3\text{-}C_6)$cycloalkyl, $(C_6\text{-}C_{18})$aryl, $(C_6\text{-}C_{18})$aryl$(C_1\text{-}C_4)$alkyl, $(C_1\text{-}C_{12})$alkyl$(C_6\text{-}C_{18})$aryl, $(C_3\text{-}C_6)$cycloalkyl$(C_6\text{-}C_{12})$aryl, $(C_5\text{-}C_{12})$hétéroaryl including 1 to 3 heteroatoms, NR'R" and NHCOR'R", R' and R", independently from each other, being selected from the hydrogen atom and the groups $(C_1\text{-}C_6)$alkyl, $(C_3\text{-}C_6)$cycloalkyl, $(C_6\text{-}C_{12})$aryl, and the $(C_6\text{-}C_{12})$ heterocycles, aromatic or not, including 1 to 3 heteroatoms or (ii) $R_x$ and Ryform together a linear or branched hydrocarbon chain having from 2 to 6 carbon atoms, optionally including one or more double bond(s) and/or optionally interrupted by an oxygen, sulfur or nitrogen atom,
- an $OR_x$ group, wherein $R_x$ corresponds to a $(C_4\text{-}C_{20})$ saturated or unsaturated fatty acid, a monosaccharide or an amino acid;

or their cosmetically acceptable salts or complexes,
to treat or prevent hair loss resulting from a too high testosterone level.

**3.** The use according to claim 1 or 2, **characterized in that** the compound of general formula (I) is selected as honokiol or magnolol respectively of formulae (II) and (III) :

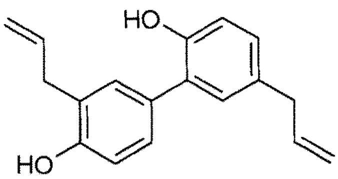

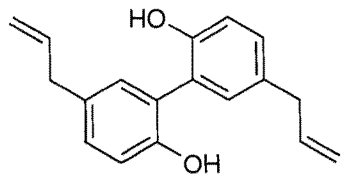

(II) (III)

**4.** The use according to any one of claims 1 to 3, **characterized in that** the compound(s) of general formula (I) is/are associated with other compounds having various properties such as mucopolysaccharides, vitamins, ceramides, anti-radical substances, U.V. filters, antioxidants and other active agents.

Figure 1

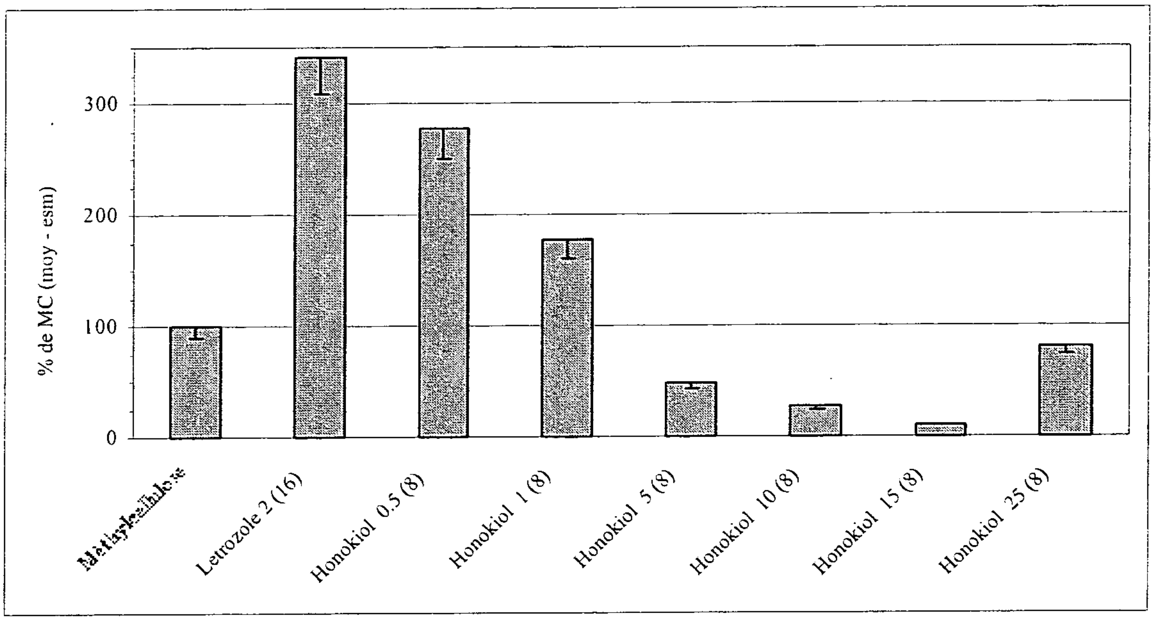

% de MC (moy - esm)
300
200
100
0
Letrozole 2 (16)
Honokiol 0.5 (8)
Honokiol 1 (8)
Honokiol 5 (8)
Honokiol 10 (8)
Honokiol 15 (8)
Honokiol 25 (8)

Figure 2

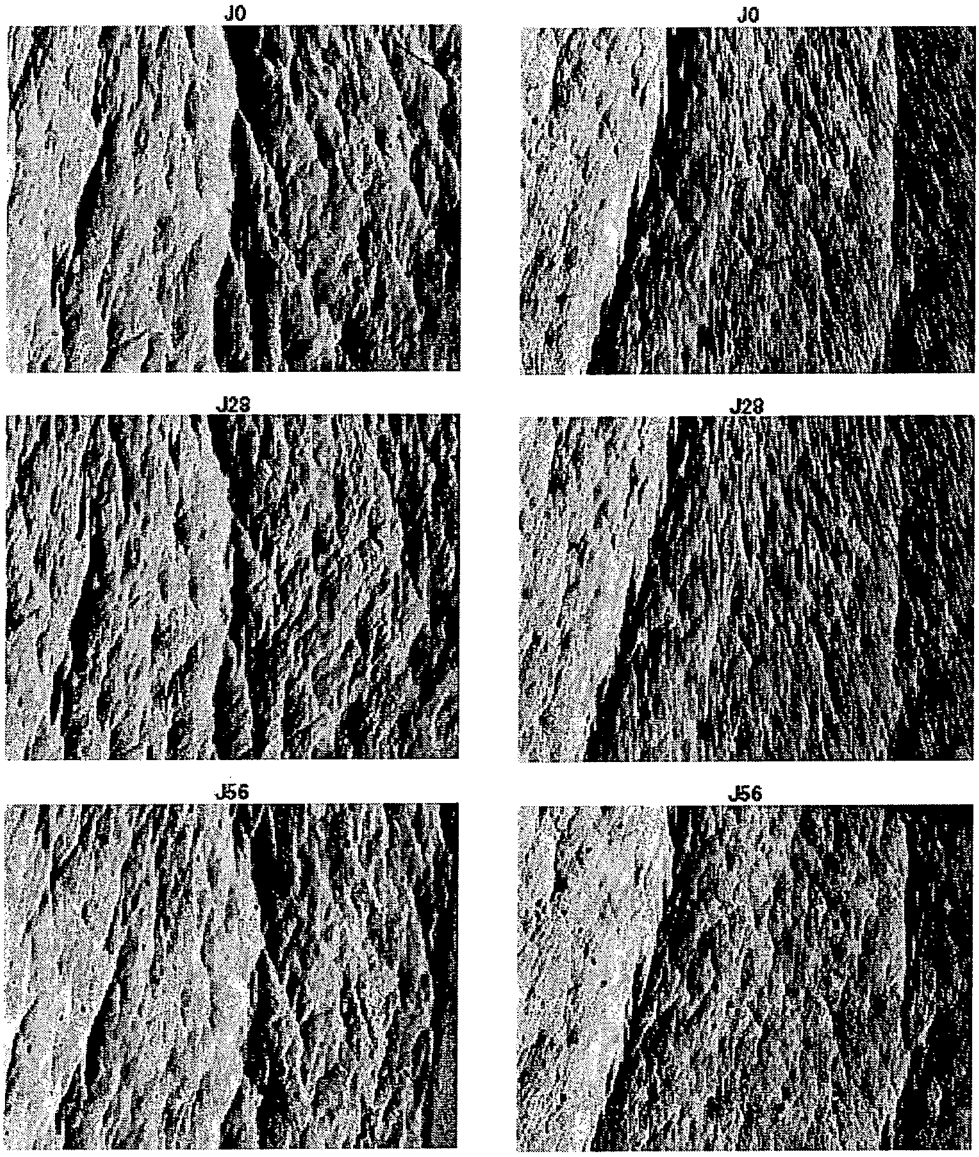

Figure 3

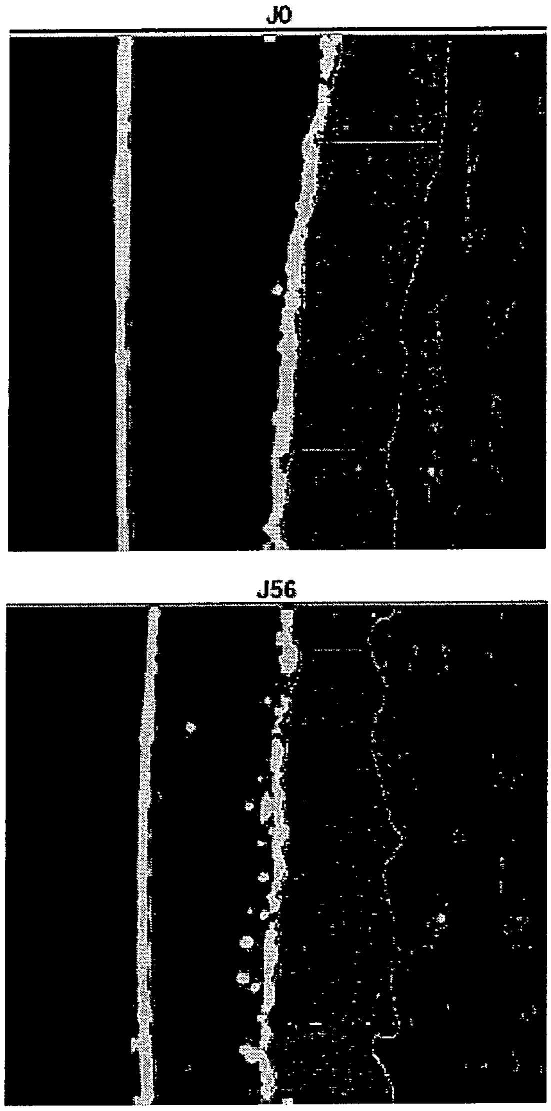

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- CN 1748673
- KR 2006014203
- US 20060140885 A
- US 6338855 B
- US 2006251608 A
- JP 4082830 A


### Littérature non-brevet citée dans la description


- IUPAC Recommendations. *Pure Applied Chemistry,* 2000, vol. 72 (8), 1493-1523